Europäisches Patentamt

European Patent Office

Office européen des brevets

(11) Veröffentlichungsnummer: **0 248 938 B1**

## (12) EUROPÄISCHE PATENTSCHRIFT

(45) Veröffentlichungstag der Patentschrift: **02.01.92**

(21) Anmeldenummer: **86108470.5**

(22) Anmeldetag: **20.06.86**

(51) Int. Cl.⁵: **B01D 35/00**, A61M 5/165, B01D 29/50, B01D 29/27

(54) **Blutfilter.**

(30) Priorität: **15.05.86 DE 8613187 U**

(43) Veröffentlichungstag der Anmeldung:
**16.12.87 Patentblatt 87/51**

(45) Bekanntmachung des Hinweises auf die
Patenterteilung:
**02.01.92 Patentblatt 92/01**

(84) Benannte Vertragsstaaten:
**AT BE CH DE FR GB IT LI LU NL SE**

(56) Entgegenhaltungen:
**EP-A- 0 024 601      EP-A- 0 079 553**
**DE-A- 3 406 928      DE-B- 1 097 087**
**DE-U- 8 534 330      FR-A- 2 305 196**

(73) Patentinhaber: **TRANSMED Medizintechnik GmbH**
**Josefstrasse 4**
**W-4798 Wünnenberg 2(DE)**

(72) Erfinder: **Alt, Erwin**
**Wesendonkstrasse 30**
**W-8000 München 81(DE)**
Erfinder: **Rappert, Klaus-Jürgen**
**Josefstrasse 4**
**W-4798 Wünnenberg 2(DE)**

(74) Vertreter: **Kehl, Günther, Dipl.-Phys. et al**
**Patentanwälte Hagemann & Kehl Ismaninger Strasse 108 Postfach 86 03 29**
**W-8000 München 86(DE)**

# Beschreibung

Die Erfindung bezieht sich auf ein Blutfilter mit einer Filterkammer, die eine Eingangsleitung und eine Auslaßleitung aufweist, und mit einem in der Filterkammer angeordneten sackförmigen Grobsieb und mindestens einem das Grobsieb umschließenden Feinsieb, wobei die Einlaßleitung dicht in den Innenraum des Grobsiebs mündet.

Blutfilter dienen dazu, bei der Transfusion Aggregate und Koageln im Blut oder Blutbestandteilen zurückzuhalten und zu verhindern, daß diese Aggregate in den Blutkreislauf des Empfängers geraten. Sie können selbstverständlich auch zur Reinigung von Infusionslösungen aller Art verwendet werden. Die Filter müssen so beschaffen sein, daß keine lebensfähigen festen Blutbestandteile in nennenswerter Weise zurückgehalten oder beschädigt werden.

Blutfilter weisen in der Regel einen mit ihrer Einlaßleitung in Verbindung stehenden Einstechdorn auf, mit dem die Blutkonserve angezapft wird. Dabei wird der Einstechdorn üblicherweise von oben in die Blutkonserve eingestochen. In dieser Lage wird die Blutkonserve gedrückt, so daß das Blut über die Einlaßleitung in die Filterkammer strömt und die Luft aus dieser verdrängt. Sobald die Luft verdrängt oder weitgehend verdrängt ist, wird die aus Blutfilter und Blutkonserve bestehende Einheit umgedreht, so daß nunmehr sich die Blutkonserve oberhalb des Filters befindet und das Blut infolge der Schwerkraft aus der Konserve in das Filter und von dort in den Körper des Patienten fließt. Es ist dabei ebenfalls möglich, die Konserve unter Druck zu halten, um den Transfusionsvorgang zu beschleunigen.

Die meisten gängigen Blutfiltertypen sind so ausgelegt, daß das Blut die verschiedenen Siebe von außen nach innen durchströmt. Es hat sich dabei gezeigt, daß bei dem Anzapfen der Blutkonserve in der oben beschriebenen Art und Weise sich die Luft aus der Filterkammer nicht vollständig entfernen läßt. Vielmehr verbleiben in der Filterkammer häufig Luftblasen, die sich an der Wandung des Siebes oder der Siebe anlegen und die für die Filtrierung des Blutes aktive Siebfläche verringern. Bei sehr großen Luftblasen kann schließlich die gesamte Transfusion blockiert werden.

Aus der DE-A-34 06 928, von der bei der Formulierung des Patentanspruches 1 ausgegangen wurde, ist ein aus kollabierbaren Flachfiltern bestehendes Blutfilter bekannt, bei dem der Einstichdorn in den inneren Siebbeutel mündet. Das bekannte Gerät gewährleistet gleichwohl keine störungsfreie Transfusion, weil insbesondere beim Wechsel einer Blutkonserve die Gefahr besteht, daß wegen des momentanen Druckabfalles die Siebe kollabieren und zusammenkleben. Dadurch wird ebenfalls die wirksame Siebfläche verkleinert und der Durchfluß entsprechend verringert. Da die Filterkammer und die Tropfkammer bei dem bekannten Blutfilter aus einem flexiblen Kunststoffschlauch gefertigt sind, besteht weiter die Gefahr, daß auch die Filterkammer kollabiert, insbesondere dann, wenn sich diese im Laufe des Transfusionsvorgangs erwärmt. Auch besteht die Gefahr, daß in der Hektik einer ersten Notversorgung mit der Hand Druck auf die flexible Filterkammer ausgeübt wird, so daß Luft, die in dem innersten Siebbeutel eingeschlossen ist, beim Zapfvorgang in die Blutkonserve gepreßt wird, wodurch der Anzapfvorgang des weiteren erschwert und zeitlich verzögert wird. Trotz der Tatsache, daß der Einstichdorn in den Innenraum des innersten Siebbeutels mündet, ist daher mit dem bekannten Blutfilter keine störungsfreie Transfusion gewährleistet.

Aus der DE-U-8 534 330 ist ein Filterbauteil für Tropfkammern von Transfusionsgeräten bekannt, das ein längliches, korbähnliches Filter aufweist. Das Bauteil ist an seinem oberen Ende mit einem flanschartigen Paßsitz versehen. Bei dem bekannten Filterbauteil ist nur eine einzige Filterstufe, bestehend aus einem einzigen korbförmigen Filter vorgesehen. Es stellen sich somit dort keine Probleme, die sich aus dem Einschluß von Luft zwischen zwei Siebflächen oder aus der gegenseitigen Berührung dieser Siebflächen ergeben könnten. Andererseits hat jedoch das Filterbauteil wegen der einstufigen Ausführung nur eine beschränkte Filterkapazität, d.h. die Siebfläche ist bereits nach der Transfusion einer vergleichsweisen geringen Blutmenge verstopft. Gemessen an dieser geringen Filterkapazität hat es andererseits ein sehr großes Füllvolumen.

Der Erfindug liegt die Aufgabe zugrunde, ein Blutfilter der eingangs genannten Art zu schaffen, das eine möglichst störungsfreie Transfusion ermöglicht, bei der insbesondere während des gesamten Transfusionsvorgangs die vorhandene Siebfläche möglichst vollständig ausgenutzt wird.

Die Lösung dieser Aufgabe wird dadurch erreicht, daß die Filterkammer eine starre Gehäusewandung aufweist, und daß die Siebe Stützgerüste mit jeweils zwei um 180° versetzt angeordneten Längsstegen aufweisen, die in radialer Richtung nach innen und nach außen über die Siebfläche vorstehen.

Durch die Kombination der Maßnahmen gemäß der Erfindung wird sichergestellt, daß sich beim ersten Anfüllen des Blutfilters keine Luft zwischen den verschiedenen Siebflächen fängt und auf diese Weise die aktive Filterfläche verringert. Beim Betrieb des Filters ist gewährleistet, daß auch bei einem Druckabfall infolge Unterbrechung der Blutströmung keine Störungen, wie beispielsweise Zusammenkleben von Siebflächen, ereignen können.

Dadurch, daß die Längsstege des Stützgerüsts in radialer Richtung nach innen und außen über die Siebfläche vorstehen, sorgt das Stützgerüst nicht nur für eine Beibehaltung der Form der sackförmigen Siebe, sondern gewährleistet, daß diese einen gegenseitigen Abstand einhalten, ohne daß durch Abstandstücke ein Teil der Siebfläche abgedeckt und somit die wirksame Siebfläche verringert wird.

In herstellungstechnischer Hinsicht hat sich eine Ausführungsform als besonders vorteilhaft erwiesen, bei der das Grobsieb mit einem Kunststoffkragen versehen ist, mit dem es auf einen mit der Einlaßleitung in Verbindung stehenden, in die Filterkammer vorstehenden Anschlußstutzen dicht aufsteckbar ist. Auf diese Weise ergibt sich eine einfache, jedoch ausreichend sichere Verbindung.

Der Außenrand des Kragens des Grobsiebes kann nach einer weiteren vorteilhaften Ausführungsform der Erfindung wiederum so ausgestaltet sein, daß auf ihn der Kunststoffkragen des nächstfolgenden Feinsiebes in gleicher Weise aufsteckbar ist. Auf diese Weise können mehrere Siebe mit geringem technischem Aufwand in Kaskade geschaltet werden.

Nach einer besonders vorteilhaften Weiterbildung der Erfindung kann dabei durch das Vorsehen von Einkerbungen und dazu passenden Vorsprüngen in den Kunststoffkrägen sichergestellt werden, daß die Siebe jeweils um 90° versetzt zusammensteckbar sind. Damit sind auch die aus den beiden Längsstegen bestehenden Stützgerüste gegeneinander versetzt, so daß sich bei geringstmöglichem gerätetechnischem Aufwand eine optimale, stabile gegenseitige Abstützung und Halterung der einzelnen Siebe einstellt. Durch die Art der Befestigung der Siebe wird desweiteren erreicht, daß die einzelnen Siebe sehr nahe ohne Gefahr einer gegenseitigen Berührung beieinanderliegend angeordnet werden können, so daß die Filterkammer entsprechend klein ausgelegt werden kann.

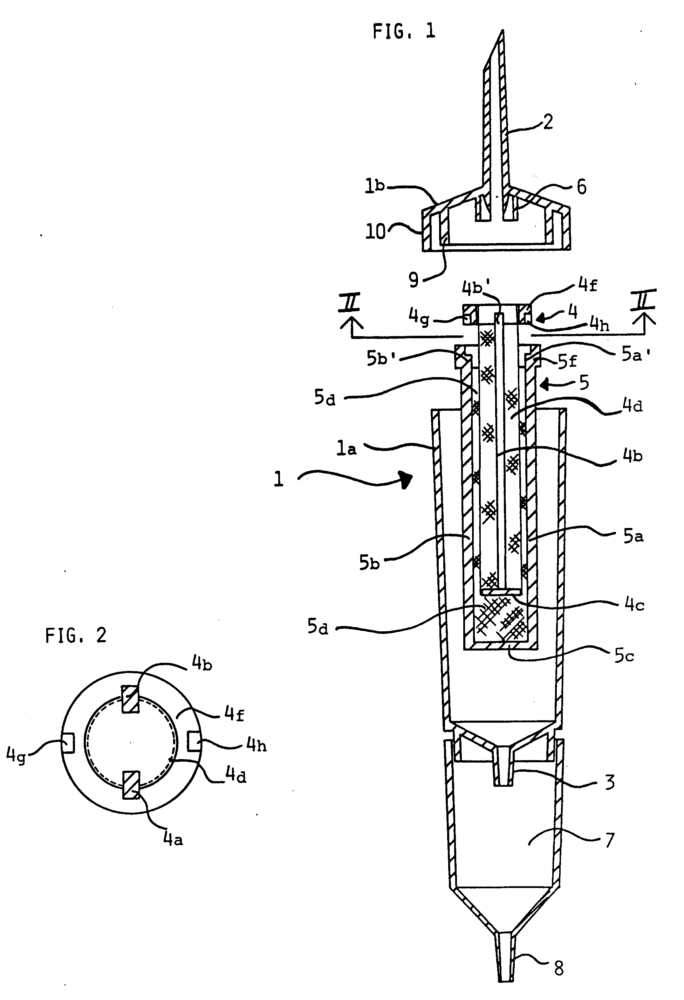

Die Erfindung wird nachfolgend anhand des in den Figuren 1 und 2 schematisch dargestellten Ausführungsbeispieles näher erläutert. Es zeigt:

Figur 1: Ein Blutfilter im Längsschnitt, teilweise nach Art einer Explosionsdarstellung auseinander gezogen,

Figur 2: einen Schnitt längs der Schnittlinie II-II, in vergrößertem Maßstab.

In Figur I ist ein Blutfilter zu erkennen, das eine zweistufige Filterkaskade aufweist. Das Blutfilter weist eine Filterkammer I auf, die aus einem Deckel Ib und einem rohrförmigen Gehäuse Ia besteht. Das Gehäuse Ia besteht aus starrem, transparentem Material und ist leicht konisch. An seiner unteren Seite geht es in einen Auslauftrichter 3 über. Der Auslauftrichter 3 ragt in eine auf das untere Ende der Filterkammer I aufgesteckte und dort dicht verklebte Tropfkammer 7. In der Tropfkammer 7 stellt sich bei der Transfusion ein bestimmter Blutpegel ein. Das Blut läuft über einen Ablaufstutzen 8, auf den ein Transfusionsschlauch aufgesteckt ist, ab. Der Kammerdeckel Ib weist zwei etwa zylindrische, konzentrische Ansätze 9 und Io auf, die den oberen Rand des Gehäuses Ia umschließen. Der Deckel Ib ist mittels Klebstoff fest mit dem oberen Rand des Gehäuses Ia verbunden. Der Kammerdeckel weist einen zentralen Einstechdorn 2 auf, über den die Verbindung zu einer Blutkonserve hergestellt werden kann. Koaxial zum Einstechdorn 2 ist ein Anschlußstutzen 6 angeordnet, dessen Außenmantel zwei um 180° versetzte Längsschlitze aufweist.

Auf den Anschlußstutzen 6 ist ein Grobsieb 4 dicht aufgesteckt. Das Grobsieb 4 weist einen ringförigen Kunststoffkragen 4f auf, dessen lichte Weite dem Außendurchmesser des Anschlußstutzens 6 entspricht, so daß es dicht auf diesen aufgeschoben werden kann. Der Kunststoffkragen 4f weist zwei Einkerbungen 4g und 4h auf, die auch insbesondere in der Schnittdarstellung der Figur 2 zu erkennen sind. An dem Kunststoffkragen sind zwei Längsstege 4a und 4b angeschweißt (siehe auch Fig. 2). Die Längsstege 4a und 4b ragen über die Innenfläche des Kragens vor. Beim Aufstecken auf den Anschlußstutzen 6 laufen die Enden der Stege 4a und 4b in die Längsschlitze des Anschlußstutzens 6 ein. Die Längsstege 4a und 4b tragen an ihrem, dem Kunststoffkragen 4f abgewandten Ende, eine etwa kreisförmige Platte 4c. Der Kunststoffkragen 4f, die Längsstege 4a und 4b sowie die Platte 4c stellen eine Stützgerüst für ein Siebgewebe 4d dar. Das von dem Stützgerüst getragene Siebgewebe besteht aus zwei Teilen, die sich jeweils von einem Längssteg 4a bis zu dem anderen Längssteg 4b erstrecken. In Längsrichtung erstrecken sich die beiden Siebgewebe von dem Kragen 4f bis zu der Abschlußplatte 4c. Das Siebgewebe 4d ist an den Längsstegen 4a und 4b so angeschweißt, daß die Längsstege nach innen und nach außen über die Ebene der Siebfläche vorstehen.

Das Grobsieb 4 wird von einem Feinsieb 5 umgeben. Das Feinsieb 5 ist in gleicher Weise wie das Grobsieb 4 aufgebaut, mit dem Unterschied, daß die Porengröße seines Siebgewebes 5d kleiner ist, und daß die sonstigen äußeren Abmessungen größer sind, so daß es das Grobsieb in sich aufnehmen kann.

Insbesondere ist der Kunststoffkragen 5f des Feinsiebes so bemessen, daß seine lichte Weite dem Außenumfang des Kragens 4f des Grobsiebes entspricht, so daß die beiden Krägen 4f und 5f ineinander gesteckt werden können. Ein Ineinanderstecken der beiden Siebe ist jedoch nur dann möglich, wenn die Längsstege 5a und 5b des Feinsiebes 5 um die Längsachse um 90° gegen die Längsstege 4a und 4b des Grobsiebes verdreht werden. In der Darstellung der Figur I sind die Längsstege 5a und 5b des Feinsiebes 5 in der Zeichenebene dargestellt, während die Längsstege 4a und 4b des Grobsiebes 4 in einer Ebene senkrecht zur Zeichenebene liegen. Bei dieser Art der winkelmäßig versetzen Zusammenfügung der Siebe laufen die nach innen vorstehenden Enden der Stege 5a und 5b in die Einkerbungen 4g und 4h des Kragens 4f des Grobfilters ein. Bei jeder anderen Positionierung stoßen dagegen die vorstehenden Längsstege 5a und 5b an dem Außenumfang des Kragens 4f des Grobsiebes 4 an, so daß die beiden Kragen nicht ineinander gesteckt werden können. Durch die winkelmäßig versetzte Ineianderanordnung der beiden Siebe und insbesondere durch die Tatsache, daß die Längsstege radial sowohl nach innen als auch nach außen über die Ebene der Siebfläche vorstehen, wird stets die Einhaltung eines Mindestabstandes zwischen dem Siebgewebe des Grobsiebes und dem Siebgewebe des Feinsiebes gewährleistet. Ansonsten entspricht der Aufbau des Feinsiebes dem Aufbau des Grobsiebes. Insbesondere entspricht die Stützplatte 5c des Feinsiebes der Stützplatte 4c des Grobsiebes.

Wenngleich in dem Ausführungsbeispiel nur zwei Siebe vorgesehen sind, kann selbstverständlich ein weiteres Feinsieb über dem Feinsieb 5 angeordnet werden, und zwar in gleicher Weise, wie das Feinsieb 5 über dem Grobsieb 4 angeordnet ist. Ersichtlich kann somit eine drei-, vier- oder sogar mehrstufige Filterkaskade hergestellt werden.

Das Blutfilter gemäß der Neuerung arbeitet wie folgt:
Der Einstechdorn 2 wird von oben, d. h. in einer Lage, die zu der in Figur I gezeigten Lage "auf den Kopf gestellt" ist, in eine Blutkonserve eingestochen. Die Blutkonserve wird komprimiert, so daß das Blut in der Bohrung des Einstechdornes 2 aufsteigt und über den Anschlußstutzen 6 in das Innere des Grobsiebes 4 eingeleitet wird. Das Blut strömt durch das Siebgewebe des Grobsiebes in den Innenraum des Feinsiebes und tritt mit ansteigendem Pegel in die Filterkammer I ein, während die Luft in dem Grobsieb 4 wie auch in dem Feinsieb 5 durch die Siebgewebe ungehindert nach außen in die Filterkammer I austreten kann. Von dort strömt die Luft über den Auslauftrichter 3 und die Tropfkammer 7 sowie über einen an dem Anschlußstutzen 8 der Tropfkammer angeschlossenen Transfusionsschlauch (nicht dargestellt) ab. Das Einschließen von Luftblasen ist nicht möglich. Wenn die Luft vollständig aus der Filterkammer I verdrängt ist, wird der Transfusionsschlauch (meist mit einer Rollenklemme) abgeklemmt. Das Gerät wird dann um 180° gedreht, so daß es die in Figur I gezeigte Position einnimmt. Dann strömt Blut aus der Konserve infolge der Schwerkraft durch den Einstichdorn 2 in den Innenraum des Grobsiebes 4 und durch dessen Gewebe in den Innenraum des Feinsiebes 5. Es verläßt das Feinsieb durch dessen Gewebe und strömt in der Filterkammer nach unten zu dem Auslauftrichter 3. Es verläßt die Tropfkammer 7, die etwa zu einem Drittel mit Blut gefüllt ist, über den Stutzen 8 und strömt über den Transfusionsschlauch in den Körper des Patienten. Die Strömungsgeschwindigkeit kann durch Ausübung von Druck auf die Blutkonserve erhöht werden.

**Patentansprüche**

1. Blutfilter mit einer Filterkammer (1), die eine Einlaßleitung (2) und eine Auslaßleitung (3) aufweist, und mit einem in der Filterkammer (1) angeordneten sackförmigen Grobsieb (4) und mindestens einem, das Grobsieb (4) umschließenden Feinsieb (5), wobei die Einlaßleitung dicht in den Innenraum des Grobsiebes (4) mündet, dadurch gekennzeichnet, daß die Filterkammer eine starre Gehäusewandung aufweist, und daß die Siebe Stützgerüste (4a, 4b, 4c; 5a, 5b, 5c) mit jeweils zwei um 180° versetzt angeordneten Längsstegen (4a, 4b, 5a, 5b) aufweisen, die in radialer Richtung nach innen und nach außen über die Siebflächen (4d, 5d) vorstehen.

2. Blutfilter nach Anspruch 1, dadurch gekennzeichnet, daß das Grobsieb (4) einen Kragen (4f) aufweist, mit dem es auf einen mit der Einlaßleitung (2) in Verbindung stehenden, in das Kammerinnere vorstehenden Anschlußstutzen (6) dicht aufsteckbar ist.

3. Blutfilter nach Anspruch 1, dadurch gekennzeichnet, daß das Feinsieb oder die Feinsiebe (5) jeweils einen Kragen (5f) aufweisen, der auf den Kragen (4f) des Grobsiebes (4) oder den Kragen des Feinsiebes mit dem nächstkleineren Durchmesser aufsteckbar ist.

4. Blutfilter nach Anspruch 3, dadurch gekennzeichnet, daß der Kragen (4f, 5f) eines jeden Siebes (4,5) Einkerbungen (g, h) und dazu um 90° versetzt angeordnete Vorsprünge (4b', 5a', 5b') aufweist, die beim Ineinanderstecken der Siebe (4,5) zusammenwirken, so daß zwei aufeinanderfolgende Siebe jeweils um 90° versetzt sind.

5. Blutfilter nach Anspruch 4, dadurch gekennzeichnet, daß die Vorsprünge (4b', 5a', 5b') an der Innenseite des Kragens (4f, 5f) angeordnet sind, und aus den Enden der Längsstege (4a, 4b, 5a, 5b) des Stützgerüstes bestehen, und daß sich die Einkerbungen (4g, 4h) an der Außenseite des Kragens (4f, 5f) befinden.

## Claims

1. Blood filter with a filter chamber (1), which has an inlet line (2) and an outlet line (3), and with a sack-shaped coarse filter (4) disposed in the filter chamber (1) and at least one fine filter (5) surrounding the coarse filter (4), the inlet line discharging in a sealed manner into the interior of the coarse filter (4), characterised in that the filter chamber has a rigid housing wall and that the filters have supporting frames (4a, 4b, 4c; 5a, 5b, 5c) with two respective longitudinal webs (4a, 4b, 5a, 5b) arranged offset by 180° which extend inwardly in the radial direction and outwardly beyond the sieve surfaces (4d, 5d).

2. Blood filter as claimed in Claim 1, characterised in that the coarse filter (4) has a collar (4f) with which it may be pushed in a sealed manner onto a connecting socket (6) in communication with the inlet line (2) and projecting into the interior of the chamber.

3. Blood filter as claimed in Claim 1, characterised in that the fine filter or the fine filters (5) have a respective collar (5f) which may be pushed onto the collar (4f) of the coarse filter (4) or the collar of the fine filter with the next smallest diameter.

4. Blood filter as claimed in Claim 3, characterised in that the collar (4f, 5f) of each filter (4, 5) has notches (g, h) and projections (4b', 5a', 5b') disposed offset therefrom by 90°, which cooperate when the filters are pushed into one another so that two successive filters are offset by 90°.

5. Blood filter as claimed in Claim 4, characterised in that the projections (4b', 5a', 5b') are arranged on the inner side of the collar (4f, 5f) and constitute the ends of the longitudinal webs (4a, 4b, 5a, 5b) of the supporting frame and that the notches (4g, 4h) are situated at the outer side of the collar (4f, 5f).

## Revendications

1. Filtre à sang comportant une chambre de filtrage (1) pourvue d'une canalisation d'entrée (2) et d'une canalisation de sortie (3), un tamis gros (4) en forme de sac placé dans la chambre de filtrage (1) et au minimum un tamis fin (5) entourant le tamis gros (4), la canalisation d'entrée débouchant de manière étanche à l'intérieur du tamis gros (4), caractérisé en ce que la chambre de filtrage présente une paroi de bâti rigide et en ce que les tamis présentent des charpentes support (4a,4b,4c;5a,5b,5c) comportant respectivement deux barrettes longitudinales (4a,4b,5a,5b) décalées de 180°, qui dépassent en direction radiale, vers l'intérieur et vers l'extérieur, des surfaces des tamis (4d, 5d).

2. Filtre à sang selon la revendication 1, caractérisé en ce que le tamis gros (4) présente une collerette (4f), au moyen de laquelle il peut s'emboîter de manière étanche sur un raccord (6) communiquant avec la canalisation d'entrée (2) et faisant saillie à l'intérieur de la chambre.

3. Filtre à sang selon la revendication 1, caractérisé en ce que le ou les tamis fin(s) (5) présente(nt) chacun une collerette (5f), qui peut s'emboîter sur la collerette (4f) du tamis gros (4) ou la collerette du tamis fin de diamètre immédiatement inférieur.

4. Filtre à sang selon la revendication 3, caractérisé en ce que la collerette (4f,5f) de chacun des tamis (4,5) présente des entailles (g,h) ainsi qu'en outre des saillies (4b',5a',5b') décalées de 90°, qui coopèrent lors de l'emboîtement des tamis (4,5) entre eux, de sorte que deux tamis successifs sont respectivement décalés de 90°.

5. Filtre à sang selon la revendication 4, caractérisé en ce que les saillies (4b',5a',5b') sont placées sur la face interne de la collerette (4f,5f) et sont constituées par les extrémités des barrettes longitudinales (4a,4b,5a,5b) de la charpente support et en ce que les entailles (4g,4h) se trouvent sur la face externe de la collerette (4f,5f).

FIG. 1

FIG. 2